# EUROPÄISCHE PATENTSCHRIFT

(11) **EP 0 550 866 B1**
(45) Veröffentlichungstag und Bekanntmachung des Hinweises auf die Patenterteilung: **09.10.1996**
(21) Anmeldenummer: 92121705.5
(22) Anmeldetag: 21.12.1992
(51) Int. Cl.: C07B 37/04, C07C 15/46, C07C 25/28, C07C 43/215, C07C 2/86, C07C 17/26, C07C 41/30

(54) **Verfahren zur Herstellung von vinylsubstituierten Aromatem aus Arylaminen**
Process for the preparation of vinyl-substituted aromatics from arylamines
Procédé pour la préparation de composés aromatiques vinyl-substitués

(30) Priorität: 24.12.1991 DE 4143021
(43) Veröffentlichungstag der Anmeldung: 14.07.1993
(73) Patentinhaber: HOECHST AKTIENGESELLSCHAFT, 65926 Frankfurt am Main (DE)
(72) Erfinder: Beller, Matthias, Dr., W-6272 Niedernhausen (DE); Strutz, Heinz, Dr., W-6230 Frankfurt am Main 80 (DE)

(56) Entgegenhaltungen:
- JOURNAL OF ORGANIC CHEMISTRY. Bd. 46, Nr. 24, 20. November 1981, EASTON US Seiten 4885 - 4888 K. KIKUKAWA ET AL 'PALLADIUM(0)-CATALYZED ARYLATION OF OLEFINS BY ARYLAMINES AND AN ALKYL NITRITE'
- BULLETIN OF THE CHEMICAL SOCIETY OF JAPAN Bd. 52, Nr. 9, September 1979, TOKYO JP Seiten 2609 - 2610 K. KIKUKAWA ET AL 'REACTION OF DIAZONIUM SALTS WITH TRANSITION METALS. II. PALLADIUM-CATALYZED ARYLATION OF ETHYLENE WITH ARENEDIAZONIUM SALTS'
- TETRAHEDRON, (INCL. TETRAHEDRON REPORTS) Bd. 37, Nr. 1, 1981, OXFORD GB Seiten 31 - 36 K. KIKUKAWA ET AL 'REACTION OF DIAZONIUM SALTS WITH TRANSITION METALS-III. PALLADIUM(0)-CATALYZED ARYLATION OF UNSATURATED COMPOUNDS WITH ARENEDIAZONIUM SALTS'

## Beschreibung

Die vorliegende Erfindung betrifft ein Verfahren Zur Herstellung von vinylsubstituierten Aromaten durch katalytische Arylierung von Ethylen mit Arylaminen.

Vinylsubstituierte Aromaten sind wichtige Monomere für die Synthese von Polymeren und stellen bedeutende Zwischenprodukte zur Herstellung von Pharmazeutika und Agrochemikalien dar.

Die üblichen Methoden zur Herstellung von Styrol und seinen Derivaten, wie die Dehydrierung von Alkylaromaten oder die Eliminierung der entsprechenden Alkohole, verlaufen meist unter drastischen Reaktionsbedingungen, zeigen oftmals unzureichende Selektivitäten oder gehen von schwer zugänglichen Edukten aus, und sind daher für die technische Herstellung von vielen funktionalisierten Styrolen nicht geeignet.

Es ist bekannt, daß Diazoniumsalze mit Ethylen unter Druck in Gegenwart von Palladiumbisbenzylidenkatalysatoren Styrole bilden (Bull. Chem. Soc. Jpn. 52, (1979), S. 2609; Tetrahedron 37 (1981), S. 31). Dabei ist es aber notwendig, mit einem großen Überschuß an basischen Salzen und unter Druck (6 bis 8 atm Ethylen) zu arbeiten. Außerdem müssen die einzusetzenden Diazoniumsalze zunächst aus den entsprechenden Aminen separat hergestellt werden, was technisch nachteilig ist.

Es ist weiter bekannt, Arylamine in Gegenwart von Säuren und tert.-Butylnitrit mit flüssig vorliegenden Olefinen palladiumkatalysiert umzusetzen (Chem. Lett. (1977), S. 159; J. Org. Chem. 46 (1981), S. 4885). Bei diesem Verfahren werden in der Regel Gemische von Säuren, wie Chloressigsäure und Essigsäure als Lösungsmittel verwendet. Die beschriebenen Reaktionstemperaturen von 50°C sind für empfindliche Substrate, wie funkionalisierte Styrole wenig geeignet. Als Ausgangsverbindungen werden reaktive α,β-ungesättigte Carbonylverbindungen, wie Acrylester, und hochsiedende nicht aktive Olefine, wie Cyclohexen oder 1-Hexen und Styrol beschrieben. Das in J. Org. Chem. 46 (1981), S. 4885 beschriebene Verfahren ergibt dabei für nichtaktivierte Olefine geringere Ausbeuten als für aktivierte Alkene. Die Herstellung von vinylsubstituierten Aromaten wird nicht beschrieben.

Arylierungen von Olefinen mit Arylaminen und tert.- Butylnitrit unter Zusatz von Palladium(II)acetat wurden auch in J. Org. Chem. 45 (1980), S. 2359 erwähnt. Dabei werden jedoch stöchiometrische Mengen der teuren Palladiumverbindung verwendet, und die Umsetzung gelingt nicht katalytisch. Auch sind die Ausbeuten nicht befriedigend. Diese Methode ist daher für die technische Herstellung größerer Substanzmengen ungeeignet.

Allen beschriebenen Methoden ist außerdem gemeinsam, daß sie unter typischen metallorganischen Schutzgasbedingungen (Stickstoff- oder Argon-Inertatmosphäre) durchgeführt werden, was zusätzlichen apparativen Aufwand bedeutet, und eine technische Realisierung problematisch macht.

Aufgabe der vorliegenden Erfindung war es nun, ein Verfahren zur katalytischen Herstellung von vinylsubstituierten Aromaten durch Arylierung von Ethylen mit Arylaminen bereitzustellen, das die vorstehend beschriebenen Nachteile nicht besitzt und das insbesondere die vinylsubstituierten Aromaten in möglichst hoher Ausbeute ohne störende Verunreinigungen und in einfacher Weise liefert.

Die Lösung dieser Aufgabe gelingt erfindungsgemäß überraschenderweise durch ein Verfahren zur Herstellung von vinylsubstituierten Aromaten der Formel (I) Ar-CH = CH₂, worin Ar eine Phenylgruppe, die 1 bis 5 Substituenten tragen kann, Naphthyl oder Anthryl oder einen 1 bis 7 Substituenten tragenden Naphthyl- oder einen 1 bis 8 Substituenten tragenden Anthrylrest, oder eine einkernige fünf-, sechs- oder siebengliedrige, heterocyclische aromatische Gruppe mit einem Sauerstoffatom und/oder einem oder zwei Stickstoffatomen im Ring, die 1 bis 5 Substituenten tragen kann, darstellt, das dadurch gekennzeichnet ist, daß man ein Arylamin der Formel (II) Ar-NH₂, worin Ar die genannte Bedeutung hat, in einer organischen Säure mit einem organischen Nitrit der Formel (III) R-ONO, worin R eine Alkylgruppe mit 1 bis 18 C-Atomen, einen Phenylrest, der mit 1 bis 3 Alkylgruppen mit jeweils 1 bis 4 C-Atomen substituiert sein kann, oder eine Alkylcarbonylgruppe mit 2 bis 5 C-Atomen darstellt, und Ethylen in einem organischen Lösungsmittel in Gegenwart einer Palladium(0)- oder Palladium(II)-Verbindung bei Temperaturen von 0 bis 35°C umsetzt.

Die Substituenten im Rest Ar sind gleich oder verschieden und stellen vorzugsweise einen nicht-aromatischen Kohlenwasserstoff mit 1 bis 8 C-Atomen, insbesondere mit 1 bis 4 C-Atomen, besonders bevorzugt jedoch Methyl, einen Alkoxy- oder Alkylthiorest mit jeweils 1 bis 8 C-Atomen, Aryl oder Aryloxy mit jeweils 6 bis 14 C-Atomen, eine -OCOR-, -COOR-, -COR-, -OSiR₃-, -NHCOR-, -NR₂-, -PR₂-, -PO₂R-, -PO₃R-, -SOR-, -SO₂R- oder -SO₃R-Gruppe , wobei R jeweils Wasserstoff, Alkyl mit 1 bis 12 C-Atomen, bevorzugt mit 1 bis 6 C-Atomen, besonders bevorzugt mit 1 bis 4 C-Atomen, und/oder Aryl mit 6-10 C-Atomen bedeutet, oder eine -OH-, -CH-, -NO-, oder -NO₂-Gruppe oder Halogen, insbesondere Fluor, Chlor oder Brom, dar. Der Begriff Alkyl soll dabei jeweils auch Cycloalkyl mit umfassen.

Als organische Nitrite der Formel (III) können beispielsweise Methyl-, Ethyl-, n- oder i-Propyl-, die verschiedenen Butyl-, Pentyl-, Hexyl- oder Octylnitrite, Benzoylnitrit, Phenylnitrit, o-, m-, p-Methylphenylnitrit, Acetylnitrit und Propionylnitrit eingesetzt werden, wobei auch Gemische verschiedener organischer Nitrite in Frage kommen. Bevorzugt handelt es sich um Alkylnitrite, wie tert.-Butyl- oder Amylnitrit. Bezogen auf das Arylamin(II) wird das Nitrit zweckmäßig in der 0,5 bis 10fachen, vorzugsweise der 0,7 bis 5fachen, besonders bevorzugt der 1 bis 2fachen, molaren Menge eingesetzt.

Als Verbindungen der Formel (II) kommen beispielsweise die folgenden in Frage: Anilin, das in o-, m- oder p-Stellung mit Methyl, Ethyl, n-Propyl, i-Propyl, n-Butyl,i-Butyl, tert.-Butyl, Fluor, Chlor, Brom, Jod, Hydroxy, Benzyloxy oder Acetoxy substituiert sein kann, sowie Aminoacetophenon, Aminoanthrachinon, Aminoazobenzol, Aminobenzamid, 2-Aminobenzimidazol, Aminobenzoesäure, Aminobenzolphosphonsäure, Aminobenzotrifluorid, Aminobenzylalkohol, Aminobiphenyl, 4-Amino-2-chlorbenzoesäure, p-Anisidin, 2-Amino-3,5-dichlorpyridin, Aminodiphenylether, 2-Amino-4-methoxy-5-nitroanisol, Aminomethylbenzoesäure, Aminomethylbenzylalkohol, Aminophenylboronsäure, Aminophenylessigsäure, Aminophthalsäure, Aminopyrimidin und trans-4-Aminozimtsäure Hydrochlorid. Bevorzugte Verbindungen der Formel (II) sind Aminomethylbenzol, Aminomethoxybenzol, Aminoacetoxybenzol, Aminonitrobenzol, Aminonitrotoluol, Aminobenzoesäure, Diaminobenzol, Aminopyrimidin, Aminomethylacetoxybenzol, p-Aminopropionsäurephenylester, Aminobenzoesäureethylester und Aminobenzoesäurenitril.

Bei der organischen Säure handelt es sich im allgemeinen um Ameisensäure, Essigsäure, Propionsäure, Trifluoressigsäure oder Chloressigsäure. Bevorzugt sind Ameisensäure, Propionsäure und Chloressigsäure, insbesondere jedoch Essigsäure. Im allgemeinen wird die Säure, bezogen auf das Arylamin (II) in der 0,1 bis 1000fachen, vorzugsweise in der 1 bis 100fachen, besonders bevorzugt in der 10 bis 50fachen, Gewichtsmenge eingesetzt.

Bei dem organischen Lösungsmittel handelt es sich im allgemeinen um aprotische und protische organische Lösungsmittel, mit Ausnahme von Carbonsäuren. Das organische Lösungsmittel wird zweckmäßig in der 0,1 bis 500fachen, insbesondere in der 1 bis 100fachen Gewichtsmenge, bezogen auf das Arylamin (II), zugesetzt. Geeignete organische Lösungsmittel sind beispielsweise gegebenenfalls chlorierte aliphatische, cycloaliphatische oder aromatische Kohlenwasserstoffe, wie n-Pentan, n-Heptan, n-Octan, Cyclopentan, Cyclohexan, Benzol, Toluol, Xylole und Chlorbenzol; aromatische, aliphatische und cyclische Ether, wie Anisol, Diethylether, Di-isopropylether, Tetrahydrofuran, Methyl-tert.-butylether und Dioxan; N-substituierte Morpholine, wie N-Methyl- und N-Formylmorpholin; Nitrile, besonders Benzonitril und Alkylnitrile mit 2 bis 5C-Atomen, wie Propionitril und Butyronitril; 3-Methoxypropionitril und 3-Ethoxypropionitril; Dialkylsulfoxide` wie Dimethyl- und Diethylsulfoxid; N,N-Dialkylamide von aliphatischen Monocarbonsäuren mit 1 bis 3 C-Atomen im Säureteil, wie N,N-Dimethylformamid und N,N-Dimethylacetamid; Alkohole mit bis zu 8 C-Atomen, wie Ethanol, n-Propanol und tert.-Butanol; aliphatische und cyclische Ketone, wie Aceton, Diethylketon, Methylisopropylketon, Cyclopentanon, Cyclohexanon, 1,3-Dimethyl-2-imidazolidinon und 1,3-Dimethyl-3,4,5,6-tetrahydro-2-(1H)-pyrimidinon; Tetramethylharnstoff; Ester, wie Ester der Kohlensäure, z.B. Diethylcarbonat, Nitromethan; Alkyl- oder Alkoxyalkylester von aliphatischen Monocarbonsäuren mit insgesamt 2 bis 8 C-Atomen, wie Essigsäuremethyl-, -ethyl-, -n-butyl- und -isobutylester, Buttersäureethyl- und -n-butylester sowie 1-Acetoxy-2-ethoxyethan und 1-Acetoxy-2-methoxyether oder Triethylphosphat. Bevorzugte Lösungsmittel sind jedoch chlorierte aliphatische Kohlenwasserstoffe, besonders Dichlormethan und Chloroform, und Diethylether, tert.-Butylmethylether, Tetrahydrofuran, Dioxan, Ethanol, n-Propanol, Acetonitril und tert.-Butanol.

Der Palladiumkatalysator wird, bezogen auf das Arylamin (II) zweckmäßig in der 0,0001 bis 1fachen, vorzugsweise in der 0,001 bis 0,1fachen, insbesondere in der 0,01 bis 0,08fachen, molaren Menge eingesetzt. Dabei handelt es sich beispielsweise um Palladium(II)acetat, Palladium(II)-chlorid, Palladium(II)nitrat, Tetrakis(triarylphosphin)palladium, Palladium(II)trifluoracetat, Palladium(II)bromid, Dinatriumtetrachloropalladat, Bisdibenzylidenpalladium(0) oder Trisdibenzylidendipalladium. Diese können beispielsweise auf heterogenen Trägern, wie Aktivkohle, Aluminiumoxid, Bariumsulfat und Calciumcarbonat aufgebracht sein, sie können aber auch direkt als Feststoffe oder aber als Lösungen in den genannten Lösungsmitteln eingesetzt werden.

Bevorzugt kommen jedoch Palladium(II)acetat und Palladium(0) auf Aktivkohle als Katalysatoren zum Einsatz.

Das erfindungsgemäße Verfahren wird vorzugsweise bei Temperaturen von 15 bis 30°C, insbesondere bei 20 bis 25°C, durchgeführt. Bei höheren Temperaturen sinkt die Aktivität des Katalysators und es kommt zu Nebenreaktionen. Besonders unreaktive Verbindungen können jedoch auch bei höheren Temperaturen noch zufriedenstellend umgesetzt werden.

Der Druck, bei dem das Ethylen zudosiert bzw. die Reaktion durchgeführt wird, ist bei dem erfindungsgemäßen Verfahren unkritisch. Es ist jedoch von Vorteil, Ethylen oder ein Gemisch von Ethylen mit inerten Gasen, wie Stickstoff, Argon oder niedere Alkane, beispielsweise Ethan, bei Atmosphärendruck durch die Reaktionslösung oder über die Reaktionslösung hinweg zu leiten. Die Reaktion kann aber auch bei Ethylenüberdruck bzw. unter Überdruck des Gasgemisches durchgeführt werden. Der Druck beträgt dabei 1 bis 500 bar.

Nach erfolgter Reaktion wird die Reaktionsmischung nach üblichen Verfahren, wie beispielsweise in J. Org. Chem. 46 (1981), S. 4885 beschrieben, aufgearbeitet.

Das erfindungsgemäße Verfahren ist insofern besonders überraschend, da Versuche, vinylsubstituierte Aromaten analog zu dem in J. Org. Chem. 46 (1981) S. 4885 publizierten Verfahren herzustellen, lediglich unbefriedigende Ausbeuten ergaben. So sind nach diesem Verfahren bei 50°C und ohne den Zusatz eines organischen Lösungsmittels vinylsubstituierte Aromaten nur in einer Ausbeute von unter 7 % erhältlich (vgl. Vergleichsversuch 1). Durch die Erniedrigung der Reaktionstemperatur (Vergleichsversuch 2) oder durch den Zusatz eines organischen Lösungsmittels läßt sich die Ausbeute jeweils geringfügig verbessern. Überraschenderweise kommt es jedoch durch die Kombination dieser beiden Reaktionsparameter zu einer synergistischen Verbesserung der Ausbeute an den gewünschten Produkten.
Weiterhin bilden sich nach dem in J. Org. Chem. 46 (1981) S. 4885 beschriebenen Verfahren aus Arylaminen und Styrolen Stilbene in relativ hohen Ausbeuten. Überraschenderweise reagieren im Vergleich dazu Styrole im vorliegenden Verfahren nur sehr langsam, weshalb sich unerwünschte Stilbene nur in sehr geringen Mengen von unter 5 % bilden. Diese Weiterreaktion zu Stilbenen ist auch bei der Heck-Reaktion von Arylhalogeniden mit Ethylen ein Problem.

Das erfindungsgemäße Verfahren ermöglicht die Synthese von vinylsubstituierten Aromaten in einem Schritt unter besonders milden Bedingungen (niedrige Temperaturen) auf einfache und effiziente Weise (kein Schutzgas, geringer apparativer Aufwand). Die niedrigen Reaktionstemperaturen sind besonders für die Herstellung von reaktiveren Styrolen von Vorteil.

Darüberhinaus handelt es sich bei dem vorliegenden Verfahren um ein ökologisch vorbildliches Verfahren, bei dem weder Koppelprodukte noch stöchiometrische Mengen von Salzabfällen entstehen.

Im Gegensatz zur Reaktion von Diazoniumsalzen mit Ethylen (Bull. Chem. Soc. Jpn. 52 (1979), S. 2609) wird bei dem vorliegenden Verfahren auf eine Isolierung des Diazoniumsalzes verzichtet, wodurch das Verfahren technisch wesentlich einfacher durchzuführen ist. Auch wird auf den Einsatz von teuren Chemikalien wie Tetrafluoroborsäure verzichtet. Außerdem kommt es nicht zur Bildung von schwer abtrennbaren Nebenprodukten wie Arylbutenen.

Der Zusatz eines organischen Lösungsmittels ist insbesondere deshalb von Vorteil, da so die Lebensdauer des aktiven Katalysators erhöht und die Aufarbeitung der Reaktionsmischung vereinfacht wird.

### Beispiele

1. In einem 500 ml Dreihalskolben mit Rührer, Tropftrichter und Innenthermometer wurden 50 ml Essigsäure vorgelegt und mit 2 mmol Palladiumacetat versetzt. Danach wurden 40 mmol Anilin in die Lösung getropft, wobei die Innentemperatur nicht über 30°C anstieg. Dann wurde Ethylen in die Lösung eingeleitet und 45 mmol tert.-Butylnitrit zugetropft, wobei die Innentemperatur nicht über 25°C anstieg. Nach Zugabe von 40 ml Dichlormethan wurde 16 Stunden gerührt. Es ergab sich gaschromatographisch bestimmt ein Umsatz von 80 %. Die Ausbeute an Styrol betrug 69 %, bezogen auf eingesetztes Amin.
2. Beispiel 1 wurde wiederholt, jedoch wurden anstelle von Anilin 40 mmol fein zermahlenes p-Anisidin in die Lösung gegeben. Es ergab sich gaschromatographisch bestimmt ein Umsatz von 85 %. Die Ausbeute an p-Methoxysryrol betrug 72 % bezogen auf eingesetztes Amin.
3. Beispiel 1 wurde wiederholt, jedoch wurden anstelle von Anilin 40 mmol 2-Fluoranilin in die Lösung getropft. Es ergab sich gaschromatographisch bestimmt ein Umsatz von 87 %. Die Ausbeute an 2-Fluorstyrol betrug 70 % bezogen auf eingesetztes Amin.
4. Beispiel 1 wurde wiederholt, jedoch wurden anstelle von Anilin 40 mmol 4-Chloranilin in die Lösung gegeben. Es ergab sich gaschromatographisch bestimmt ein Umsatz von 84 %. Die Ausbeute an 4-Chlorstyrol betrug 72 % bezogen auf eingesetztes Amin.
5. Beispiel 1 wurde wiederholt, jedoch wurden anstelle von Anilin 40 mmol 3-Toluidin in die Lösung gegeben. Es ergab sich gaschromatographisch bestimmt ein Umsatz von 83 %. Die Ausbeute an 3-Methylstyrol betrug 66 % bezogen auf eingesetztes Amin.
6. In einem 500 ml Dreihalskolben mit Rührer, Tropftrichter und Innenthermometer wurden 20 ml Essigsäure, 20 g Chloressigsäure und 10 mmol Anilin vorgelegt und mit 0,5 mmol Bis(dibenzylidenaceton)palladium versetzt. Dann wurde Ethylen durch die Lösung geleitet. Anschließend tropfte man eine Lösung von 11 mmol tert. Butylnitrit in 10 ml Essigsäure bei Raumtemperatur innerhalb von 15-20 Minuten zur Reaktionslösung. Dann wurden 25 ml Ethanol zugegeben. Nach 16 Stunden betrug die gaschromatographisch bestimmte Ausbeute 55 %.

### Vergleichsbeispiel 1

In einem 500 ml Dreihalskolben mit Rührer, Tropftrichter und Innenthermometer wurden 20 ml Essigsäure, 20 g Chloressigsäure und 10 mmol Anilin vorgelegt und mit 0,5 mmol Bis(dibenzylidenaceton)palladium versetzt. Dann wurde Ethylen durch die Lösung geleitet. Anschließend tropfte man eine Lösung von 11 mmol tert. Butylnitrit in 10 ml Essigsäure innerhalb von 15-20 Minuten zu der auf 50°C erwärmten Reaktionslösung.
Nach 16 Stunden betrug die gaschromatographisch bestimmte Ausbeute 7 %.

### Vergleichsbeispiel 2

Es wurde wie in Vergleichsbeispiel 1 verfahren, die Lösung von tert. Butylnitrit in Essigsäure wurde jedoch bei Raumtemperatur zugetropft.
Nach 16 Stunden betrug die gaschromatographisch bestimmte Ausbeute 15 %.

## Patentansprüche

1. Verfahren zur Herstellung von vinylsubstituierten Aromaten der Formel (I) Ar-CH=CH₂, worin Ar eine Phenylgruppe, die 1 bis 5 Substituenten tragen kann, Naphthyl oder Anthryl oder einen 1 bis 7 Substituenten tragenden Naphthyl- oder einen 1 bis 8 Substituenten tragenden Anthrylrest, oder eine einkernige fünf-, sechs- oder siebengliedrige, heterocyclische aromatische Gruppe mit einem Sauerstoffatom und/oder einem oder zwei Stickstoffatomen im Ring, die 1 bis 5 Substituenten tragen kann, darstellt, dadurch gekennzeichnet, daß man ein Arylamin der Formel (II) Ar-NH₂, worin Ar die genannte Bedeutung hat, in einer organischen Säure mit einem organischen Nitrit der Formel (III) R-ONO, worin R eine Alkylgruppe mit 1 bis 18 C-Atomen, einen Phenylrest, der 1 bis 3 Alkylgruppen mit jeweils 1 bis 4 C-Atomen tragen kann, oder eine Alkylcarbonylgruppe mit 2 bis 5 C-Atomen darstellt, und Ethylen in einem organischen Lösungsmittel mit Ausnahme von Carbonsäuren in Gegenwart einer Palladium(0)- oder Palladium(II)-Verbindung als Katalysator bei Temperaturen von 0 bis 35°C umsetzt.

2. Verfahren nach Anspruch 1, dadurch gekennzeichnet, daß die Substituenten im Rest Ar gleich oder verschieden sind und einen nicht-aromatischen Kohlenwasserstoffrest mit 1 bis 8 C-Atomen, vorzugsweise mit 1 bis 4 C-Atomen, besonders bevorzugt Methyl, einen Alkoxy- oder Alkylthiorest mit jeweils 1 bis 8 C-Atomen, Aryl oder Aryloxy mit jeweils 6 bis 14 C-Atomen, eine -OCOR-, -COOR-, -COR-, -OSiR₃-, -NHCOR-, -NR₂-, -PR₂-, -PO₂R-, -PO₃R-, -SOR-, -SO₂R- oder -SO₃R-Gruppe , wobei R jeweils Wasserstoff, Alkyl mit 1 bis 12 C-Atomen, bevorzugt mit 1 bis 6 C-Atomen, besonders bevorzugt mit 1 bis 4 C-Atomen, und/oder Aryl mit 6-10 C-Atomen bedeutet, oder eine -OH-, -CH-,-NO-, oder -NO₂-Gruppe oder Halogen, insbesondere Fluor, Chlor oder Brom, darstellen.

3. Verfahren nach Anspruch 1 oder 2, dadurch gekennzeichnet, daß das organische Lösungsmittel ein chlorierter aliphatischer Kohlenwasserstoff, Diethylether, tert.-Butylmethylether, Tetrahydrofuran, Dioxan, Ethanol, n-Propanol, Acetonitril und/oder tert.-Butanol ist.

4. Verfahren nach einem oder mehreren der Ansprüche 1 bis 3, dadurch gekennzeichnet, daß das organische Lösungsmittel in der 0,1 bis 500fachen, vorzugsweise in der 1 bis 100fachen Gewichtsmenge, bezogen auf das Arylamin, eingesetzt wird.

5. Verfahren nach einem oder mehreren der Ansprüche 1 bis 4, dadurch gekennzeichnet, daß das organische Nitrit ein Alkylnitrit ist.

6. Verfahren nach Anspruch 5, dadurch gekennzeichnet, daß es sich bei dem Alkylnitrit um tert. Butyl- oder Amylnitrit handelt.

7. Verfahren nach einem oder mehreren der Ansprüche 1 bis 6, dadurch gekennzeichnet, daß das organische Nitrit in der 0,5 bis 10fachen, vorzugsweise in der 0,7 bis 5fachen, besonders bevorzugt in der 1 bis 2fachen molaren Menge, bezogen auf das Arylamin, eingesetzt wird.

8. Verfahren nach einem oder mehreren der Ansprüche 1 bis 7, dadurch gekennzeichnet, daß es sich bei dem Arylamin der Formel (II) um Aminomethylbenzol, Aminomethoxybenzol, Aminoacetoxybenzol, Aminonitrobenzol, Aminonitrotoluol, Aminobenzoesäure, Diaminobenzol, Aminopyrimidin, Aminomethylacetoxybenzol, p-Aminopropionsäurephenylester, Aminobenzoesäureethylester und Aminobenzoesäurenitril.

9. Verfahren nach einem oder mehreren der Ansprüche 1 bis 8, dadurch gekennzeichnet, daß es sich bei der organischen Säure um Ameisensäure, Essigsäure, Propionsäure oder Chloressigsäure handelt.

10. Verfahren nach einem oder mehreren der Ansprüche 1 bis 9, dadurch gekennzeichnet, daß die organische Säure in der 0,1 bis 1000fachen, vorzugsweise der 1 bis 100fachen, besonders bevorzugt der 10 bis 50fachen Gewichtsmenge, bezogen auf das Arylamin, eingesetzt wird.

11. Verfahren nach einem oder mehreren der Ansprüche 1 bis 10, dadurch gekennzeichnet, daß Palladium(II)acetat oder Palladium(0) auf Aktivkohle als Katalysatoren eingesetzt werden.

12. Verfahren nach einem oder mehreren der Ansprüche 1 bis 11, dadurch gekennzeichnet, daß der Katalysator in der 0,0001 bis 1fachen, vorzugsweise der 0,001 bis 0,1fachen, besonders bevorzugt der 0,01 bis 0,08fachen molaren Menge, bezogen auf das Arylamin, eingesetzt wird.

13. Verfahren nach einem oder mehreren der Ansprüche 1 bis 12, dadurch gekennzeichnet, daß das Verfahren bei Temperaturen von 15 bis 30°C, vorzugsweise von 20 bis 25°C, durchgeführt wird.

14. Verfahren nach einem oder mehreren der Ansprüche 1 bis 13, dadurch gekennzeichnet, daß das Ethylen allein oder im Gemisch mit inerten Gasen eingesetzt wird.

15. Verfahren nach Anspruch 14, dadurch gekennzeichnet, daß das inerte Gas Stickstoff, Argon oder ein niederes Alkan ist.

16. Verfahren nach einem oder mehreren der Ansprüche 1 bis 15, dadurch gekennzeichnet, daß das Ethylen oder das Gemisch von Ethylen mit inerten Gasen bei einem Druck von 1 bis 500 bar in die Reaktionsmischung geleitet wird.

## Claims

1. A process for preparing vinyl-substituted aromatic compounds of the formula (I) Ar-CH=CH₂, where Ar is a phenyl group that may bear 1 to 5 substituents, naphthyl or anthryl or a naphthyl radical bearing 1 to 7 substituents or an anthryl radical bearing 1 to 8 substituents, or a mononuclear five-, six- or seven-membered heterocyclic aromatic group which has an oxygen atom and/or one or two nitrogen atoms in the ring and may bear 1 to 5 substituents, which comprises reacting an arylamine of the formula (II) Ar-NH₂, where Ar has the aforementioned meaning, in an organic acid with an organic nitrite of the formula (III) R-ONO, where R is an alkyl group having 1 to 18 carbon atoms, a phenyl radical which may bear 1 to 3 alkyl groups each having 1 to 4 carbon atoms, or is an alkylcarbonyl group having 2 to 5 carbon atoms, and ethylene in an organic solvent with the exception of carboxylic acids in the presence of a palladium(0) or palladium(II) compound as catalyst at temperatures of 0 to 35°C.

2. The process as claimed in claim 1, wherein the substituents in the Ar radical are identical or different and are a non-aromatic hydrocarbon radical having I to 8 carbon atoms, preferably having 1 to 4 carbon atoms, particularly preferably methyl, an alkoxy or alkylthio radical having in each case 1 to 8 carbon atoms, aryl or aryloxy having in each case 6 to 14 carbon atoms, an -OCOR, -COOR, -COR, -OSiR₃, -NHCOR, -NR₂, -PR₂, -PO₂R, -PO₃R, -SOR, -SO₂R or -SO₃R group, where R is in each case hydrogen, alkyl having 1 to 12 carbon atoms, preferably having 1 to 6 carbon atoms, particularly preferably having 1 to 4 carbon atoms, and/or aryl having 6-10 carbon atoms, or is an -OH, -CN, -NO or -NO₂ group or halogen, in particular fluorine, chlorine or bromine.

3. The process as claimed in claim 1 or 2, wherein the organic solvent is a chlorinated aliphatic hydrocarbon, diethyl ether, tert.-butyl methyl ether, tetrahydrofuran, dioxane, ethanol, n-propanol, acetonitrile and/or tert.-butanol.

4. The process as claimed in one or more of claims 1 to 3, wherein the organic solvent is used in a 0.1 to 500 fold, preferably in a 1 to 100 fold amount by weight with respect to the arylamine.

5. The process as claimed in one or more of claims 1 to 4, wherein the organic nitrite is an alkyl nitrite.

6. The process as claimed in claim 5, wherein the alkyl nitrite is tert.-butyl or amyl nitrite.

7. The process as claimed in one or more of claims 1 to 6, wherein the organic nitrite is used in a 0.5 to 10 fold, preferably in a 0.7 to 5 fold, particularly preferably in a 1 to 2 fold molar amount with respect to the arylamine.

8. The process as claimed in one or more of claims 1 to 7, wherein the arylamine of the formula (II) is aminomethylbenzene, aminomethoxybenzene, aminoacetoxybenzene, aminonitrobenzene, aminonitrotoluene, aminobenzoic acid, diaminobenzene, aminopyrimidine, aminomethylacetoxybenzene, phenyl p-aminopropionate, ethyl aminobenzoate and amino benzonitrile.

9. The process as claimed in one or more of claims 1 to 8, wherein the organic acid is formic acid, acetic acid, propionic acid or chloroacetic acid.

10. The process as claimed in one or more of claims 1 to 9, wherein the organic acid is used in a 0.1 to 1000 fold, preferably a 1 to 100 fold, particularly preferably a 10 to 50 fold amount by weight with respect to the arylamine.

11. The process as claimed in one or more of claims 1 to 10 fold, wherein palladium(II) acetate or palladium(0) on activated charcoal are used as catalysts.

12. The process as claimed in one or more of claims 1 to 11, wherein the catalyst is used in a 0.0001 to 1 fold, preferably a 0.001 to 0.1 fold, particularly preferably a 0.01 to 0.08 fold molar amount with respect to the arylamine.

13. The process as claimed in one or more of claims 1 to 12, wherein the process is carried out at temperatures of 15 to 30°C, preferably of 20 to 25°C.

14. The process as claimed in one or more of claims 1 to 13, wherein the ethylene is used alone or in a mixture with inert gases.

15. The process as claimed in claim 14, wherein the inert gas is nitrogen, argon or a lower alkane.

16. The process as claimed in one or more of claims 1 to 15, wherein the ethylene or the mixture of ethylene with inert gases is passed into the reaction mixture at a pressure of 1 to 500 bar.

## Revendications

1. Procédé pour la préparation de composés aromatiques substitués par vinyle de formule (I) Ar-CH=CH₂, où Ar représente un groupe phényle qui peut porter 1 à 5 substituants, naphtyle ou anthryle ou un radical naphtyle portant 1 à 7 substituants, ou un radical anthryle portant 1 à 8 substituants, ou un groupe aromatique hétérocyclique mononucléé à 5, 6 ou 7 chaînons avec un atome d'oxygène et/ou un ou deux atomes d'azote dans le cycle, qui peut porter 1 à 5 substituants, caractérisé en ce que l'on fait réagir une arylamine de formule (II) Ar-NH₂, dans laquelle Ar a la signification déjà citée, dans un acide organique avec un nitrite organique de formule (III) R-ONO où R représente un groupe alkyle avec 1 à 18 atomes de carbone, un radical phényle qui peut être substitué par 1 à 3 groupes alkyle avec chaque fois 1 à 4 atomes de carbone, ou un groupe alkylcarbonyle avec 2 à 5 atomes de carbone, avec l'éthylène, dans un solvant organique les acides carboxyliques exceptés, en présence d'un composé de palladium(0) ou de palladium(II), à des températures de 0 à 35 °C.

2. Procédé selon la revendication 1, caractérisé en ce que les substituants dans le radical Ar sont identiques ou différents et représentent de préférence un hydrocarbure non aromatique avec 1 à 8 atomes de carbone, plus particulièrement avec 1 à 4 atomes de carbone, mais de manière particulièrement préférée méthyle, un radical alcoxy ou alkylthio chacun avec 1 à 8 atomes de carbone, aryle ou aryloxy chacun avec 6 à 14 atomes de carbone, un groupe -OCOR-, -COOR-, -COR-, -OSiR₃-, -NHCOR-, -NH₂-, -PR₂-, -PO₂R-, -PO₃R-, -SOR-, -SO₂R- ou -SO₃R-, R représentant chaque fois l'hydrogène, alkyle avec 1 à 12 atomes de carbone, de préférence avec 1 à 6 atomes de carbone, de manière particulièrement préférée avec 1 à 4 atomes de carbone, et/ou aryle avec 6 à 10 atomes de carbone ou un groupe -OH-, -CN-, -NO- ou -NO₂- ou halogène, plus particulièrement le fluor, le chlore ou le brome.

3. Procédé selon la revendication 1 ou 2, caractérisé en ce que le solvant organique est un hydrocarbure chloré aliphatique, l'éther diéthylique, l'éther tert-butylméthylique, le tétrahydrofuranne, le dioxanne, l'éthanol, le n-propanol, l'acétonitrile et/ou le tert-butanol.

4. Procédé selon une des revendications 1 à 3, caractérisé en ce que le solvant organique est utilisé dans une quantité en poids de 0,1 à 500 fois, de préférence de 1 à 100 fois par rapport à l'arylamine.

5. Procédé selon une ou plusieurs des revendications 1 à 4, caractérisé en ce que le nitrite organique est un nitrite d'alkyle.

6. Procédé selon la revendication 5, caractérisé en ce que dans le cas de nitrite d'alkyle, il s'agit de nitrite de tert-butyle ou de nitrite d'amyle.

7. Procédé selon une ou plusieurs des revendications 1 à 6, caractérisé en ce que le nitrite organique est utilisé dans une quantité de 0,5 à 10 fois, de préférence de 0,7 à 5 fois, de manière particulièrement préférée de 1 à 2 fois en moles par rapport à l'arylamine.

8. Procédé selon une ou plusieurs des revendications 1 à 7, caractérisé en ce que dans le cas d'arylamines de formule (II), il s'agit de l'aminométhylbenzène, de l'aminométhoxybenzène, de l'aminoacétoxybenzène, de l'aminonitrobenzène, de l'aminonitrotoluène, de l'acide aminobenzoïque, du diaminobenzène, de l'aminopyrimidine, de l'aminométhylacétoxybenzène, du p-aminopropionate de phényle, de l'aminobenzoate d'éthyle et de l'aminobenzonitrile.

9. Procédé selon une ou plusieurs des revendications 1 à 8, caractérisé en ce que dans le cas d'acides organiques, il s'agit de l'acide formique, de l'acide acétique, de l'acide propionique ou de l'acide chloracétique.

10. Procédé selon une ou plusieurs des revendications 1 à 9, caractérisé en ce que l'on utilise l'acide organique dans une quantité de 0,1 à 1000 fois, de préférence de 1 à 100 fois, de manière particulièrement préférée de 10 à 50 fois la quantité en poids par rapport à l'arylamine.

11. Procédé selon une ou plusieurs des revendications 1 à 10, caractérisé en ce que l'on utilise en tant que catalyseur de l'acétate de palladium(II) ou du palladium(0) sur du charbon actif.

12. Procédé selon une ou plusieurs des revendications 1 à 11, caractérisé en ce que le catalyseur est utilisé en une quantité de 0,0001 à 1 fois, de préférence de 0,001 à 0,1 fois, de manière particulièrement préférée de 0,01 à 0,08 fois la quantité en moles par rapport à l'arylamine.

13. Procédé selon une ou plusieurs des revendications 1 à 12, caractérisé en ce que le procédé est mis en oeuvre à des températures de 15 à 30 °C, de préférence de 20 à 25 °C.

14. Procédé selon une ou plusieurs des revendications 1 à 13, caractérisé en ce que l'on utilise l'éthylène seul ou en mélange avec des gaz inertes.

15. Procédé selon la revendication 14, caractérisé en ce que les gaz inertes sont l'azote, l'argon ou un alcane inférieur.

16. Procédé selon une ou plusieurs des revendications 1 à 15, caractérisé en ce que l'on fait barboter l'éthylène ou le mélange d'éthylène avec des gaz inertes sous une pression de 1 à 500 bars dans le mélange réactionnel.
